# EUROPEAN PATENT APPLICATION

(11) **EP 4 825 417 A1**
(43) Date of publication of application: **21.10.2026**
(21) Application number: 25305573.5
(22) Date of filing: 18.04.2025
(51) Int. Cl.: A61M 5/31

(54) **GRIP ELEMENT FOR AN INJECTION DEVICE**

(71) Applicant: Becton Dickinson France, 38800 Le Pont-de-Claix (FR)
(72) Inventor: CHIA Yu Leng, Neville, 120110 Singapore (SG)
(74) Representative: Germain Maureau

(57) **Abstract**

A grip element (10) comprising a body (20) extending between a proximal end (22) and a distal end (24) along an axis (A-A), the body including an outer surface (26) and an inner surface (28) extending between the proximal end and the distal end of the body, the outer surface of the body being configured to be engaged by a user between the proximal end and the distal end of the body and the inner surface being configured to engage at least a portion of an injection device between the proximal end and the distal end of the body; and a flange (50, 52) extending radially outward from the proximal end of the body.

## Description

### FIELD

The present disclosure relates to the field of injection devices in medical and/or cosmetic injection procedures. Particularly, the present disclosure relates to a grip element configured for use on an injection device in a medical and/or injection procedure.

### BACKGROUND

An injection device configured to deliver a material contained in the injection device to a user by injection, such as a medical injection device, a cosmetic injection device, and/or an ear, nose, and throat spray device, may include a grip element configured to be coupled to the injection device to improve handling of the injection device by a user.

Currently available grip elements are typically configured to be handled by a user in a medical injection procedure and/or a cosmetic injection procedure performed by the user, in which the user pushes a plunger element of the injection device axially in a first direction, while pulling on the grip element axially in a second direction, to dispense the material from the injection device. Accordingly, currently available grip elements are typically configured to receive an axial force exerted by the user.

In a medical injection procedure and a cosmetic injection procedure, a user may need to aspirate the injection device to determine whether a tip of the injection device has penetrated a blood vessel. Aspiration of the injection device includes the user pulling the plunger element axially in the second direction. In a cosmetic injection procedure, a user may need to support the injection device between two or more fingers of a hand of the user during a rest period between injections, over an extended amount of time. Further, in a cosmetic injection procedure, a user may need increased control and/or accuracy due to injecting a material of a higher viscosity, such as a dermal filler (e.g. hyaluronic acid, collagen, transplanted tissue, and/or biosynthetic polymer) and/or the like, into connective tissue of a patient. Aspirating the injection device, supporting the injection device during rest periods, and increasing control and/or accuracy of the injection device may require the user to squeeze the barrel of the injection device. However, squeezing the barrel of the injection device may result in reduced control of the injection device, reduced comfortability in handling the injection device, and/or breakage of the injection device.

It is desirable to provide a grip element configured for use on an injection device that is configured to be utilized in an injection position, an aspiration position, and a rest position, during one or more of a medical procedure and a cosmetic injection procedure, in a manner that improves ergonomics, control, and safety of handling of the injection device.

### SUMMARY

According to aspects of the disclosure, a grip element configured for use on an injection device is provided. The injection device includes at least a barrel configured to contain a material, a finger support extending from the barrel, and a plunger element configured to be received within the barrel. The grip element includes a body extending between a proximal end and a distal end along an axis. The body includes an outer surface and an inner surface extending between the proximal end and the distal end of the body. The outer surface of the body is configured to be engaged by a user between the proximal end and the distal end of the body and the inner surface is configured to engage at least a portion of the injection device between the proximal end and the distal end of the body. The grip element includes a flange extending radially outward from the proximal end of the body.

According to aspects of the disclosure, the body may be configured to receive a radial force exerted by the user when the body is engaged by the user.

According to aspects of the disclosure, the body may include an elongated central portion between the proximal end and the distal end of the body.

According to aspects of the disclosure, the body may extend to a length within a range of 15mm and 40mm.

According to aspects of the disclosure, the body may be substantially tubular in shape.

According to aspects of the disclosure, the outer surface of the body may include a pad configured to be engaged by the user.

According to aspects of the disclosure, the body may be formed from a first material and the pad may be formed from a second material.

According to aspects of the disclosure, the first material of the body may include a first rigidity, the second material of the pad may include a second rigidity, and the first rigidity may be greater than the second rigidity.

According to aspects of the disclosure, the pad may be formed from an elastically deformable material.

According to aspects of the disclosure, the grip element may include a rim extending radially outward from the distal end of the body.

According to aspects of the disclosure, the inner surface of the body may define a cavity configured to receive at least a portion of the injection device.

According to aspects of the disclosure, the cavity may include a first section configured to receive at least a portion of the barrel of the injection device, a second section configured to receive at least a portion of the finger support of the injection device, and a third section configured to receive at least a portion of the plunger element of the injection device.

According to aspects of the disclosure, the body may define a first opening at or adjacent to the proximal end of the body, a second opening at or adjacent to the distal end of the body, and each of the first opening and the second opening may be in communication with the cavity.

According to aspects of the disclosure, an injection device is provided. The injection device includes a barrel configured to contain a material, a finger support extending from the barrel, a plunger element configured to be received within the barrel, and the grip element according to any aspect of the disclosure presented herein.

According to aspects of the disclosure, a method of handling an injection device is provided. The method includes providing the grip element according to any aspect of the disclosure presented herein, mounting the grip element to the injection device, and positioning the grip element within a hand of a user.

In the manner described and according to aspects illustrated herein, the grip element, the injection device, and the method are configured to be utilized in an injection position, an aspiration position, and a rest position, during one or more of a medical procedure and a cosmetic injection procedure, in a manner that improves ergonomics, control, and safety of handling of the injection device.

### BRIEF DESCRIPTION OF THE DRAWINGS

Aspects of an example will be described with reference to the drawings, where like numerals represent like parts:
Figure 1 shows a front view of a grip element configured for use on an injection device according to aspects of the present disclosure;
Figure 2 shows a front view the injection device according to Figure 1 according to aspects of the disclosure;
Figure 3 shows a perspective view of the grip element according to Figure 1 according to aspects of the disclosure;
Figure 4 shows a front view of the grip element according to Figure 1 according to aspects of the disclosure; and
Figure 5 shows a front view of an alternative configuration of the grip element according to Figure 1 according to aspects of the disclosure.

### DETAILED DESCRIPTION

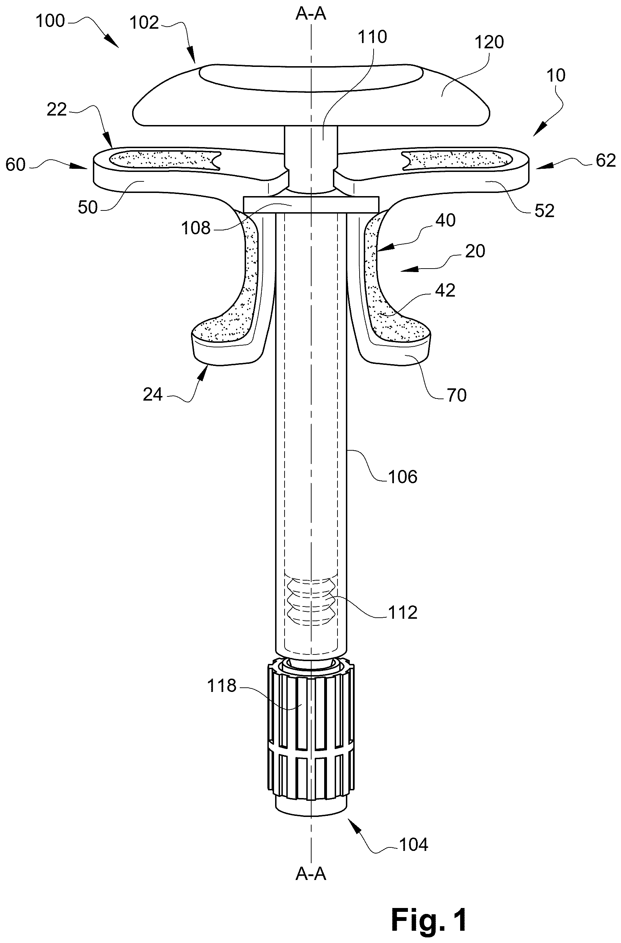

A grip element configured for use on an injection device according to aspects of the disclosure is described with reference to Figures 1-5. Like numerals represent like parts, and the grip element will generally be referred to by the reference numeral 10. Although the grip element 10 is described with reference to specific examples, it should be understood that modifications and changes may be made to these examples without going beyond the general scope as defined by the claims. In particular, individual characteristics of the various examples shown and/or mentioned herein may be combined in additional examples. Consequently, the description and the drawings should be considered in a sense that is illustrative rather than restrictive. The Figures, which are not necessarily to scale, depict illustrative aspects and are not intended to limit the scope of the disclosure. The illustrative aspects depicted are intended only as exemplary.

The term "exemplary" is used in the sense of "example," rather than "ideal." While aspects of the disclosure are amenable to various modifications and alternative forms, specifics thereof have been shown by way of example in the drawings and will be described in detail. It should be understood, however, that the intention is not to limit aspects of the disclosure to a particular example described. On the contrary, the intention of this disclosure is to cover all modifications, equivalents, and alternatives falling within the scope of the disclosure.

Various materials, methods of construction, methods of fastening, and the like may be described in the context of disclosed examples. Those skilled in the art will recognize known substitutes for the materials, construction methods, fastening methods, and the like, all of which are contemplated as compatible with the disclosed example and are intended to be encompassed by the appended claims.

As used in this disclosure and the appended claims, the singular forms "a," "an," and "the" include plural referents, unless the content clearly dictates otherwise. As used in this disclosure and the appended claims, the term "or" is generally employed in a sense including "and/or," unless the content clearly dictates otherwise.

Throughout the description, including the claims, the terms "comprising a," "including a," and "having a" should be understood as being synonymous with "comprising one or more," "including one or more," and "having one or more" unless otherwise stated. In addition, any range set forth in the description, including the claims, should be understood as including its end value(s), unless otherwise stated. Specific values for described elements should be understood to be within accepted manufacturing or industry tolerances known to one of skill in the art, and any use of the terms "substantially," "approximately," and "generally" should be understood to mean falling within such accepted tolerances.

When an element or feature is referred to herein as being "on," "engaged to," "connected to," or "coupled to" another element or feature, it may be directly on, engaged, connected, or coupled to the other element or feature, or intervening elements or features may be present. In contrast, when an element or feature is referred to as being "directly on," "directly engaged to," "directly connected to," or "directly coupled to" another element or feature, there may be no intervening elements or features present. Other words used to describe the relationship between elements or features should be interpreted in a like manner (e.g., "between" versus "directly between," "adjacent" versus "directly adjacent," etc.).

Spatially relative terms, such as "top," "bottom," "middle," "inner," "outer," "beneath," "below," "lower," "above," "upper," and the like may be used herein for ease of description to describe one element or relationship of a feature to another element(s) or feature(s) as illustrated in the drawings. Spatially relative terms may be intended to encompass different orientations of a device in use or operation in addition to the orientation depicted in the drawings. For example, if the device in the drawings is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

Although the terms "first," "second," "third," etc. may be used herein to describe various elements, components, regions, layers, sections, and/or parameters, these elements, components, regions, layers, sections, and/or parameters should not be limited by these terms. These terms are only used to distinguish one element, component, region, layer, section, or parameter from another element, component, region, layer, section, or parameter. Thus, a first element, component, region, layer, section, or parameter discussed herein could be termed a second element, component, region, layer, section, or parameter without departing from the teachings of the present disclosure.

As shown by Figures 1-5, the grip element 10 is configured for use on an injection device 100. Additionally or alternatively, it is contemplated that the grip element 10 may be referred to as and/or considered to be included by the injection device 100. However, the grip element 10 will be described herein as being independent of the injection device 100, unless description of the grip element 10 being included by the injection device 100 is otherwise necessary.

Referring to Figures 1-2, the injection device 100 to which the grip element 10 is configured for use on may be configured to deliver a material (not shown) contained in the injection device 100 to a user by injecting, spraying, and/or otherwise dispensing a fluid to and/or within a body part of the user and/or a patient of the user during an injection procedure. However, the injection device 100 will be described herein as being configured to deliver the material by injecting a fluid, unless description of the injection device 100 being configured to deliver the material by spraying and/or otherwise dispensing a fluid is otherwise necessary. Accordingly, it is contemplated that the injection device 100 may be configured to be, referred to as, and/or considered to be a "syringe," a "medical device," and/or a "cosmetic device." It is contemplated that the material contained within the injection device 100 may be a fluid having one or more of medicinal and cosmetic properties.

In examples, the injection device 100 extends between a proximal end 102 and a distal end 104 along an axis A-A. Additionally or alternatively, the axis A-A of the injection device 100 may be referred to as and/or considered to be the axis A-A of the grip element 10. In examples, the axis A-A may extend substantially longitudinally. Accordingly, the axis A-A may be referred to as and/or considered to be a "longitudinal axis." It is contemplated that the terms "proximal" and "distal" as used herein may be understood as a position and/or location corresponding to a component, device, and/or a portion thereof in relation to a hand (not shown) of the user when the user is holding the grip element 10 and/or the injection device 100. In examples, the term "proximal," with respect to the grip element 10 and/or the injection device 100, may correspond to a position and/or location provided at a distance closer to the hand of the user holding the grip element 10 and/or the injection device 100 than a "distal" position and/or location. The term "distal," with respect to the grip element 10 and/or the injection device 100, may correspond to a position and/or location provided at a distance that is farther from the hand of the user holding the grip element 10 and/or the injection device 100 than a "proximal" position and/or location. Additionally or alternatively, a "distal direction" may be understood as a direction of delivery of the material from the injection device 100 during performance of the injection procedure and a "proximal direction" may be understood as a direction substantially opposite the direction of delivery of the material from the injection device 100 and/or a direction toward the hand of the user holding the grip element 10 and/or injection device 100.

In examples, the injection device 100 may include a barrel 106 (may also be referred to as and/or considered to be a "reservoir") configured to contain the material; a finger support 108 extending radially outward from the barrel 106 and configured to allow the user to handle and control the injection device 100; a plunger element 110 configured to be received within the barrel 106, to decrease a volume within the barrel 106 to push the material out of the barrel 106, and to increase a volume within the barrel 106 to draw the material into the barrel 106 and/or aspirate the injection device 100; a stopper 112 (may also be referred to as and/or considered to be a "piston") affixed to the plunger element 110 and configured to seal the volume and/or the barrel 106; a tip 114 (may also be referred to as and/or considered to be a "needle" or a "nozzle") configured to deliver the material; a hub 116 configured to connect the tip 114 to the barrel 106; and a cap element 118 (may also be referred to herein as a "shield") configured to protect and/or seal the tip 114. In examples, the plunger element 110 may include a handle member 120 at the proximal end 102 of the injection device 100 configured to be pushed and pulled by the user. In examples, one or more of the barrel 106, the finger support 108, and the hub 116 of the injection device 100 may be formed from a glass material and/or the like.

It is contemplated that the terms "outer," "outward," "inner," and "inward" may be understood in relation to the axis A-A, such that the terms "outer" and "outward" may correspond to a position and/or direction farther from and/or away from the axis A-A in contrast with an "inner" and "inward" position and/or direction and the terms "inner" and "inward" may correspond to a position and/or direction closer to and/or toward the axis A-A in contrast with an "outer" and "outward" position and/or direction.

As shown by Figures 1 and 3-5, the grip element 10 includes a body 20 extending substantially axially between a proximal end 22 and a distal end 24. It is contemplated that the proximal end 22 and the distal end 24 of the body 20 may be referred to as and/or considered to be the proximal end 22 and the distal end 24 of the grip element 10. The body 20 includes an outer surface 26 and an inner surface 28 extending between the proximal end 22 and the distal end 24 of the body 20.

Referring to Figures 1 and 3-5, the inner surface 28 of the body 20 is configured to receive the injection device 100 such that the injection device 100 may be inserted into the body 20. Additionally or alternatively, the inner surface 28 of the body 20 is configured to engage the injection device 100. In examples, the inner surface 28 of the body 20 may include a coupling member (not shown) configured to engage the injection device 100 through an interference-fit connection, a snap-fit connection, and/or the like. Accordingly, the grip element 10 is configured to be mounted to the injection device 100.

Additionally or alternatively, it is contemplated that the grip element 10 may be formed on and/or included integrally on the injection device 100. Additionally or alternatively, it is contemplated that the grip element 10 may be configured to be, referred to as, and/or considered to be the finger support 108 of the injection device 100, such that the grip element 10 is configured to replace and/or be interchangeable with a corresponding finger support 108 of a corresponding injection device 100. However, the grip element 10 will be described herein as being configured to engage the injection device 100, unless description of the grip element 10 being configured to be formed on and/or included integrally on the injection device 100 is otherwise necessary.

In examples, the inner surface 28 of the body 20 defines a first opening 30 at or adjacent to the proximal end 22 of the body 20 and a second opening 32 at or adjacent to the distal end 24 of the body 20. The first opening 30 and the second opening 32 of the body 20 are oriented about the axis A-A. Additionally, the inner surface 28 of the body 20 defines a cavity 34 extending substantially axially between the first opening 30 and the second opening 32 of the body 20. The cavity 34 of the body 20 is configured to be in communication with the first opening 30 and the second opening 32 of the body 20. Additionally or alternatively, it is contemplated that the first opening 30 and the second opening 32 of the body 20 may be configured to be, referred to as, and/or considered to be included by the cavity 34.

Each of the first opening 30, the second opening 32, and the cavity 34 of the body 20 are configured to receive at least a portion of the injection device 100. In examples, the first opening 30 of the body 20 may be configured and/or shaped to receive and/or engage at least a portion of the plunger element 110 when the injection device 100 is inserted into the body 20. Additionally, the second opening 32 may be configured and/or shaped to receive and/or engage at least a portion of the barrel 106 of the injection device 100 when the injection device 100 is inserted into the body 20. Additionally, the cavity 34 may be configured and/or shaped to receive and/or engage at least a portion of one or more of the barrel 106 of the injection device 100, the finger support 108 of the injection device 100, and the plunger element 110 of the injection device 100. In examples, the cavity 34 may include one or more of a first section 34a configured and/or shaped to receive and/or engage at least a portion of the barrel 106 of the injection device 100, a second section 34b configured and/or shaped to receive and/or engage at least a portion of the finger support 108 of the injection device 100, and a third section 34c configured and/or shaped to receive and/or engage at least a portion of the plunger element 110 of the injection device 100.

Each of the first opening 30, the second opening 32, and the cavity 34 of the body 20 may extend radially through and/or open to a clearance 36 at a face 38 (may also be referred to herein as a "side," "front," and/or "rear") of the body 20. It is contemplated that the term "face" may be understood in relation to the axis A-A, such that the term "face" may correspond to a position and/or direction perpendicular to the axis A-A. Accordingly, each of the first opening 30, the second opening 32, and the cavity 34 of the body 20 may be configured to receive at least a portion of the injection device 100 through the clearance 36 at the face 38 of the body 20, so that the injection device 100 may be inserted into the grip element 10 through the face 38 of the body 20. Additionally or alternatively, each of the first opening 30, the second opening 32, and the cavity 34 of the body 20 may be configured to receive the injection device 100 through one or more of the first opening 30 and the second opening 32 of the body 20, such that the injection device 100 may be inserted into the grip element 10 through one or more of proximal end 22 and the distal end 24 of the body 20. However, the grip element 10 will be described herein as being configured to receive the injection device 100 through the face 38 of the body 20, unless description of the grip element 10 being configured to receive the injection device 100 through one or more of the proximal end 22 and the distal end 24 of the body 20 is otherwise necessary.

When the grip element 10 is mounted to the injection device 100 and/or the injection device 100 is inserted into the grip element 10, at least a portion of the plunger element 110 of the injection device 100 extends beyond the proximal end 22 of the body 20 and at least a portion of the barrel 106 of the injection device 100 and the tip 114 of the injection device 100 extend beyond the distal end 24 of the body 20. **In** this manner, the plunger element 110 of the injection device 100 may be handled by the user beyond the proximal end 22 of the body 20 and the tip 114 of the injection device 100 may be used to deliver the material.

Referring to Figures 1 and 3-5, the outer surface 26 of the body 20 is configured to be engaged by the user. In particular, the outer surface 26 of the body 20 is configured to be engaged by the user between the proximal end 22 and the distal end 24 of the body 20. In examples, the outer surface 26 of the body 20 is configured to be engaged by at least a portion of one or more finger (not shown) of a hand of the user and/or a palm (not shown) of the hand of the user. In examples, the outer surface 26 of the body 20 may be configured to be engaged by one or more of a first finger of the hand of the user (may also be referred to as and/or considered to be an "index finger"), a second finger of the hand of the user (may also be referred to as and/or considered to be a "middle finger"), a third finger of the hand of the user (may also be referred to as and/or considered to be a "ring finger"), and the palm of the hand of the user. It is contemplated that the first finger of the hand of the user is oriented adjacent to the second finger of the hand of the user and that the second finger of the hand of the user is oriented adjacent to the third finger of the hand of the user. Additionally or alternatively, it is contemplated that the second finger of the hand of the user is oriented between the first finger and the third finger of the hand of the user.

The outer surface 26 of the body 20 is configured to receive a radial force exerted by the user when the body 20 is engaged by the user. In examples, the body 20 is configured to extend between one or more of the first finger and the second finger of the hand of the user and the second finger and the third finger of the hand of the user. In examples, the body 20 is elongated between the proximal end 22 and the distal end 24 of the body 20. The body 20 may include an elongated central portion 40 extending between the proximal end 22 and the distal end 24 of the body 20. Additionally or alternatively, the body 20 may be substantially tubular in shape. In examples, the body 20 extends to a length within a range of 15mm and 40mm and, in particular, within a range of 18mm and 32mm.

In this manner, the body 20 is configured to facilitate a first position of the grip element 10 (may also be referred to as and/or considered to be a "rest position") during the injection procedure. In the first position of the grip element 10, the body 20 is configured to allow the user to rest the grip element 10 between one or more of the first finger and the second finger and the second finger and the third finger of the hand of the user. Additionally or alternatively, in this manner, the body 20 is configured to facilitate a second position of the grip element 10 (may also be referred to as and/or considered to be an "aspiration position") during the injection procedure. In the second position of the grip element 10, the body 20 is configured to allow the user to exert a radial force on and/or to squeeze the grip element 10 between one or more of the first finger and the second finger and the second finger and the third finger of the hand of the user, thereby allowing the user to pull the plunger element 110 in the proximal direction. Accordingly, in this manner, the grip element 10 is configured to improve ergonomics, control, and safety of the injection device 100

In examples, the outer surface 26 of the body 20 may include a pad 42 configured to be engaged by the user. The pad 42 may extend between the proximal end 22 and the distal end 24 of the body 20 and/or throughout the body 20 toward and/or to the proximal end 22 and the distal end 24 of the body 20. The pad 42 may extend circumferentially about and/or throughout a circumference of the body 20. In examples, the body 20 may be formed from a first material and the pad 42 may be formed of a second material. The first material of the body 20 may include a first rigidity and the pad 42 of the body 20 may include a second rigidity. The first rigidity of the first material may be greater than the second rigidity of the second material. In examples, the body 20 may be formed from a durable material, such as a plastic material, a metal material, and/or the like. In examples, the pad 42 may be formed from an elastically deformable material, such as a rubber material, a silicone material, and/or the like. In this manner, the pad 42 is configured to enhance the improved ergonomics, control, and safety of the grip element 10.

Referring to Figures 1 and 3-5, the outer surface 26 of the body 20 is configured to receive an axial force exerted by the user when the body 20 is engaged by the user. The grip element 10 includes one or more flange 50, 52 extending radially outward from the proximal end 22 of the body 20. The flange 50, 52 is configured to be engaged by the user to receive an axial force exerted by the user on the outer surface 26 of the body 20. In examples, the flange 50, 52 includes a proximal surface 54 and a distal surface 56. The proximal surface 54 of the flange 50, 52 may be configured to receive an axial force exerted by the user in the distal direction. The distal surface 56 of the flange 50, 52 may be configured to receive an axial force exerted by the user in the proximal direction. It is contemplated that the flange 50, 52 may be configured to be, referred to as, and/or considered to be included by the body 20. Accordingly, the outer surface 26 of the body 20 extends to and/or throughout the flange 50, 52. Additionally or alternatively, the outer surface 26 of the body 20 may be referred to as and/or considered to be the outer surface 26 of the flange 50, 52.

In examples, the grip element 10 includes at least a first flange 50 included on a first side 60 of the body 20 and a second flange 52 included on a second side 62 of the body 20. It is contemplated that the term "side" may be understood in relation to the axis A-A, such that the term "side" may correspond to a position and/or direction perpendicular to the axis A-A. In examples, the first side 60 of the body 20 opposes the second side 62 of the body 20 about the axis A-A. Additionally or alternatively, the face 38 of the body 20 extends between the first side 60 and the second side 62 of the body 20. Accordingly, the body 20 may be substantially T-shaped.

The distal surface 56 of the first flange 50 may be configured to be engaged by the first finger of the hand of the user and the distal surface 56 of the second flange 52 may be configured to be engaged by the second finger of the hand of the user. Additionally or alternatively, the distal surface 56 of the first flange 50 may be configured to be engaged by the second finger of the hand of the user and the distal surface 56 of the second flange 52 may be configured to be engaged by the third finger of the hand of the user. The proximal surface 54 of one or more of the first flange 50 and the second flange 52 may be configured to be engaged by the palm of the hand of the user. In this manner, the first flange 50 and the second flange 52 are configured to facilitate a third position of the grip element 10 (may also be referred to as and/or considered to be an "injection position") during the injection procedure. In the third position of the grip element 10, the first flange 50 and the second flange 42 are configured to allow the user to exert an axial force and/or to pull the first flange 50 and the second flange 52, thereby allowing the user to push the plunger element 110 in the distal direction. Accordingly, in this manner the grip element 10 is configured to improve ergonomics, control, and safety of the injection device 100.

Referring to Figures 1 and 3-5, the grip element 10 may include a rim 70 extending radially outward from the distal end 24 of the body 20. Accordingly, the grip element 10 may be configured to receive one or more of the first finger, the second finger, and the third finger of the hand of the user between the first flange 50 and the second flange 52 and the rim 70. The rim 70 may be configured to be engaged by the user to receive an axial force exerted by the user on the outer surface 26 of the body 20. The rim 70 may be configured to receive an axial force exerted by the user in the distal direction. In particular, the rim 70 may be configured to receive an axial force exerted by one or more of the first finger and the second finger of the hand of the user and the second finger and the third finger of the hand of the user. It is contemplated that the rim 70 may be configured to be, referred to as, and/or considered to be included by the body 20. Accordingly, the outer surface 26 of the body 20 may extend to and/or throughout the rim 70. Additionally or alternatively, the outer surface 26 of the body 20 may be referred to as and/or considered to be the outer surface 26 of the rim 70. In this manner, the body 20 is configured to enhance the improved ergonomics, control, and safety of the injection device 100.

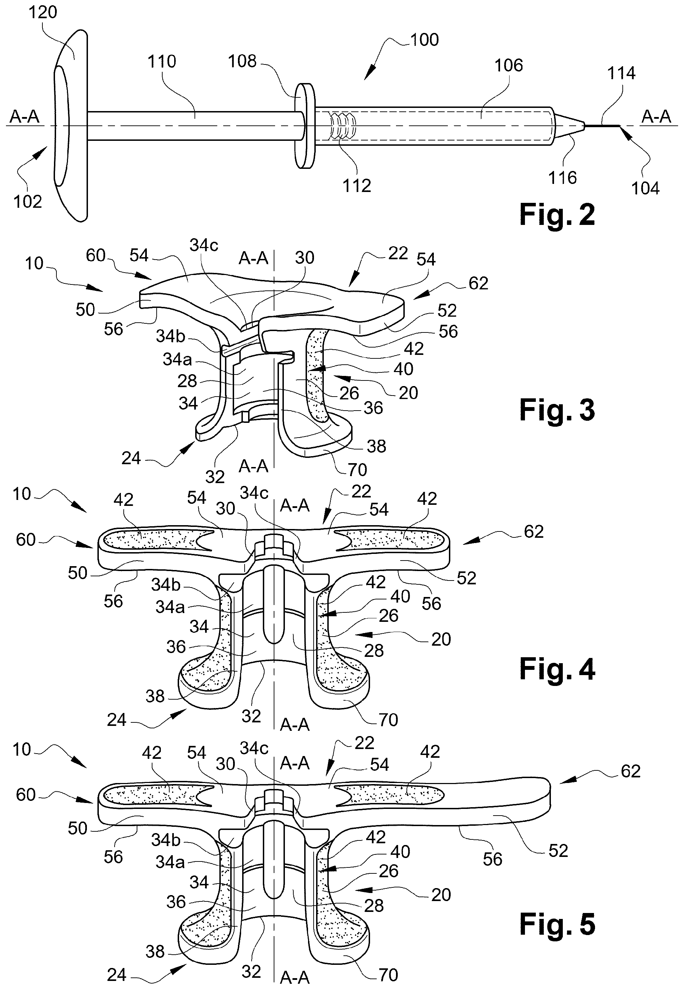

Referring to Figures 4-5, the pad 42 may extend to, throughout, and/or be included on the distal surface 56 of the first flange 50 and the distal surface 56 of the second flange 52. Additionally or alternatively, the pad 42 of the body 20 may extend to, throughout, and/or be included on the proximal surface 54 of the first flange 50 and the proximal surface 54 of the second flange 52. Additionally or alternatively, the pad 42 may extend to, throughout, and/or be included on the rim 70. In this manner, the pad 42 is configured to enhance the improved ergonomics, control, and safety of the grip element 10.

In examples, the first flange 50 may extend to a first length and the second flange 52 may extend to a second length. Referring to Figure 4, in examples, the first length of the first flange 50 may be substantially equal to the second length of the second flange 52. In examples, the first length of the first flange 50 and the second length of the second flange 52 may be within a range of 5mm and 40mm and, in particular, within a range of 12mm and 17mm. Alternatively, referring to Figure 5, in examples, the second length of the second flange 52 may be greater than the first length of the first flange 50. In examples, the first length of the first flange 50 may be within a range of 5mm and 20mm and, in particular, within a range of 12mm and 17mm. The second length of the second flange 52 may be within a range of 20mm and 40mm and, in particular, within a range of 23mm and 32mm. The second length of the second flange 52 being greater than the first length of the first flange 50 allows for a greater surface area and, thus, a greater point of contact, for engagement of one or more of the proximal surface 54 and the distal surface 56 of the second flange 52. Accordingly, the distal surface 56 of the second flange 52 may be configured to be engaged by the second finger and the third finger of the hand of the user during the injection procedure. Additionally or alternatively, the proximal surface 54 of the second flange may be configured to be engaged by a larger portion of the palm of the hand of the user during the injection procedure. Alternatively, in examples, it is contemplated that the first length of the first flange 50 may be greater than the second length of the second flange 52, as described in relation to the second length of the second flange 52 being greater than the first length of the first flange 50. In this manner, one or more of first flange 50 and the second flange 52 may be configured to enhance the improved ergonomics, control, and safety of the grip element 10.

In operation, the grip element 10 is mounted to the injection device 100 by inserting the injection device 100 into the cavity 34 of the grip element 10. The user grasps the grip element 10 and/or the injection device 100 and inserts the body 20 between the first finger and the second finger of the hand of the user or between the second finger and the third finger of the hand of the user, such that the grip element 10 may be utilized in one or more of the first position, the second position, and the third position of the grip element 10 during the injection procedure. **In** this manner, the grip element 10 is configured to be utilized in a rest position, an aspiration position, and an injection position during one or more of a medical injection procedure and a cosmetic injection procedure, in a manner that improves ergonomics, control, and safety of handling of the injection device 100.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

Additionally, all of the disclosed features of an apparatus may be transposed, alone or in combination, to a method and vice versa.

## Claims

1. A grip element (10) configured for use on an injection device (100), the injection device including at least a barrel (106) configured to contain a material, a finger support (108) extending from the barrel, and a plunger element (110) configured to be received within the barrel, the grip element comprising:
a body (20) extending between a proximal end (22) and a distal end (24) along an axis (A-A), the body including an outer surface (26) and an inner surface (28) extending between the proximal end and the distal end of the body, the outer surface of the body being configured to be engaged by a user between the proximal end and the distal end of the body and the inner surface being configured to engage at least a portion of the injection device between the proximal end and the distal end of the body; and
a flange (50, 52) extending radially outward from the proximal end of the body.

2. The grip element (10) according to claim 1, wherein the body (20) is configured to receive a radial force exerted by the user when the body is engaged by the user.

3. The grip element (10) according to any of claims 1-2, wherein the body (20) includes an elongated central portion (40) between the proximal end (22) and the distal end (24) of the body.

4. The grip element (10) according to claim any of claims 1-3, wherein the body (20) extends to a length within a range of 15mm and 40mm.

5. The grip element (10) according to claim any of claims 1-4, wherein the body (20) is substantially tubular in shape.

6. The grip element (10) according to claim any of claims 1-5, wherein the outer surface (26) of the body (20) includes a pad (42) configured to be engaged by the user.

7. The grip element (10) according to claim 6, wherein the body (20) is formed from a first material and the pad (42) is formed from a second material.

8. The grip element (10) according to claim 7, wherein the first material of the body (20) includes a first rigidity, the second material of the pad (42) includes a second rigidity, and the first rigidity is greater than the second rigidity.

9. The grip element (10) according to any of claims 6-8, wherein the pad (42) is formed from an elastically deformable material.

10. The grip element (10) according to any of claims 1-9, comprising a rim (70) extending radially outward from the distal end (24) of the body (20).

11. The grip element (10) according to any of claims 1-10, wherein the inner surface (28) of the body (20) defines a cavity (34) configured to receive at least a portion of the injection device (100).

12. The grip element (10) according to claim 11, wherein the cavity (34) includes a first section (34a) configured to receive at least a portion of the barrel (106) of the injection device (100), a second section (34b) configured to receive at least a portion of the finger support (108) of the injection device, and a third section (34c) configured to receive at least a portion of the plunger element (110) of the injection device.

13. The grip element (10) according to any of claims 11-12 wherein the body (20) defines a first opening (30) at or adjacent to the proximal end (22) of the body, a second opening (32) at or adjacent to the distal end (24) of the body, and each of the first opening and the second opening are in communication with the cavity (34).

14. An injection device (100) comprising:
a barrel (106) configured to contain a material;
a finger support (108) extending from the barrel;
a plunger element (110) configured to be received within the barrel; and
the grip element (10) according to any of claims 1-13.

15. A method of handling an injection device (100), the method comprising:
providing the grip element (10) according to any of claims 1-13;
mounting the grip element to the injection device; and
positioning the grip element within a hand of a user.
